# EUROPEAN PATENT SPECIFICATION

(11) **EP 0 821 969 B1**
(45) Date of publication and mention of the grant of the patent: **22.11.2006**
(21) Application number: 97100939.4
(22) Date of filing: 16.07.1993
(51) Int. Cl.: A61K 38/18, A61P 1/16

(54) **Medicinal Composition comprising TCF-II**
Arzneimittelzusammensetzung enthaltend TCF-II
Composition médicale contenant du TCF-II

(30) Priority: 16.07.1992 JP 21222792; 16.07.1992 JP 21222992; 10.08.1992 JP 23419892
(43) Date of publication of application: 04.02.1998
(62) Divisional of application: 93305602.0
(73) Proprietor: DAIICHI PHARMACEUTICAL CO., LTD., Chuo-ku, Tokyo 103-8234 (JP)
(72) Inventor: Masunaga, Hiroaki, Shimotsuga-gun, Tochigi (JP); Fujise, Nobuaki, Ishibashimachi, Shimotsuga-gun, Tochigi (JP); Higashi, Kanji, Kawagoe-shi, Saitama (JP)
(74) Representative: Wakerley, Helen Rachael

(56) References cited:
- EP-A- 0 461 560
- EP-A- 0 462 277
- EP-A- 0 539 590
- WO-A-91/03254
- US-A- 3 904 753
- US-A- 5 004 805
- CHEMICAL ABSTRACTS, vol. 117, no. 3, 20 July 1992 (1992-07-20) Columbus, Ohio, US; abstract no. 23902u, XP002084491 & J.S.RUBIN ET AL.: U.S. PAT. APPL. US 582,063 , 15 June 1991 (1991-06-15),
- J.S.RUBIN ET AL.: "A BROAD-SPECTRUM HUMAN..." PROC.NATL.ACAD.SCI.USA, vol. 88, January 1991 (1991-01), pages 415-419, XP002084488
- K.M.WEIDNER ET AL.: "EVIDENCE FOR THE IDENTITY OF..." PROC.NATL.ACAD.SCI.USA, vol. 88, August 1991 (1991-08), pages 7001-7005, XP002084489
- T.SEKI ET AL.: "ISOLATION AND EXPRESSION OF cDNA FOR DIFFERENT FORMS OF HEPATOCYTE GROWTH FACTOR FROM HUMAN LEUKOCYTE" BIOCHEM.BIOPHYS.RES.COMMUN., vol. 172, no. 1, 1990, pages 321-327, XP002084490

## Description

### Background of the Invention

This invention relates to medicinal compositions containing an effective amount of TCF-II for the treatment of liver diseases.

### Description of the Prior Art

Biologically active substances produced by human derived fibroblast cells, for example β-interferon as a tumor cytotoxic factor, have been well known.

Biologically active substances produced by fibroblast cells other than β-interferon such as a tumor cytotoxic glycoprotein called CBF in Japanese Unexamined Patent Publication No. 146293 (1983), a tumor cell proliferation inhibitor (INF) having a molecular weight of 35,000-45,000 in Japanese Unexamined Patent Publication No. 33120 (1986), a tumor proliferation factor (FNF) in Japanese Unexamined Patent Publication No. 1872 (1986), a physiologically active substance having a molecular weight of 40,000-60,000 and an isoelectric point of pH 5.0±0.5 in Japanese Unexamined Patent Publication No. 103021 (1987), and a tumor cytotoxic factor having a molecular weight of 36,000±1,000 and a specific amino acid sequence at isoelectric point of pH 10.5 or higher in Japanese Unexamined Patent Publication No. 10998 (1989), have been known. The inventors have been investigating antitumor substances derived from human fibroblast cells and found a new antitumor proteinous substance. Furthermore, the inventors successfully cloned a cDNA coding for the protein and determined its amino acid sequence. Also the usefulness of the protein was confirmed. The new antitumor protein and its gene were disclosed in the inventors' International Patent Publication No. 10651 (1990). The new antitumor protein was named TCF-II.

TCF-II has both potent antitumor activity and proliferation stimulative activity for normal cells and is a member of the HGF (hepatocyte growth factor) group. Molecular weight determination of TCF-II with SDS electrophoresis showed 78,000±2,000 or 74,000±2,000. The reduction products of TCF-II showed a common band (A chain) at 52,000±2,000, and two bands (B and C chains) at 30,000±2,000 and 26,000±2,000, respectively.

TCF-II may be applied for the regeneration of liver after the hepatectomy due to its proliferative effect for hepatocytes. This is now under investigation but no animal experiments confirming the effect or indicating use for the treatment of liver diseases have been known.

### Summary of the Invention

The inventors noticed the biological activity of TCF-II and have been investigating the use of TCF-II as an antitumor agent and diagnostic marker of the diseases.

The inventors found that TCF-II provides not only proliferation of hepatocytes but also therapeutic effects on various liver diseases. Heretofore, the therapeutic effect of TCF-II on various liver diseases had not been confirmed.

An object of the present invention is to provide a liver disease treatment agent comprising an effective ingredient of TCF- II.

Thus, the present invention relates to a novel liver disease treatment agent comprising an effective component of TCF-II; the agent provided by the present invention may be used for the treatment of liver diseases including cholestatic liver diseases.

### Brief Description of the Drawings

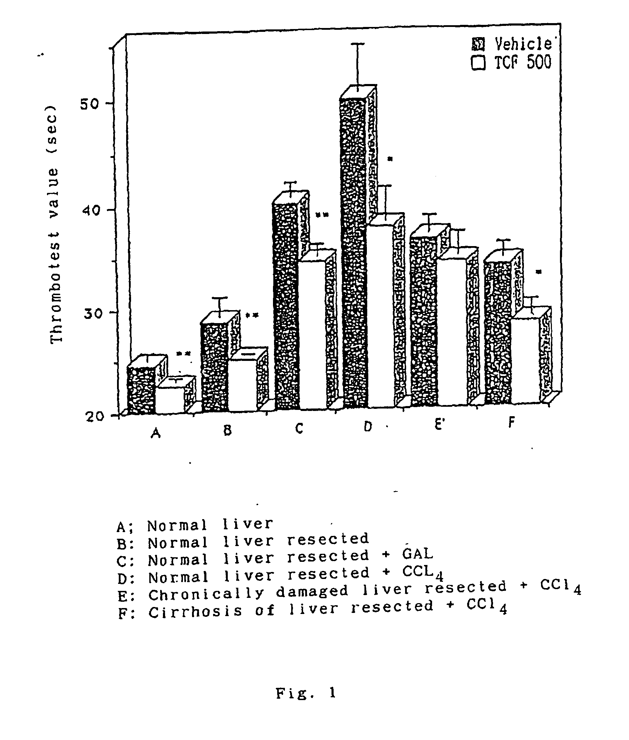

Fig. 1 shows the results of thrombotest in liver resected pathologic rats.

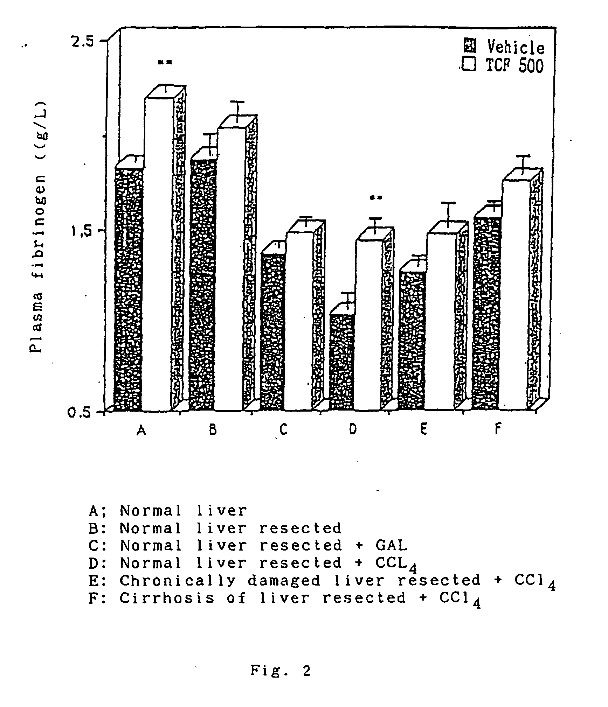

Fig. 2 shows the serum fibrinogen level in liver resected pathologic rats.

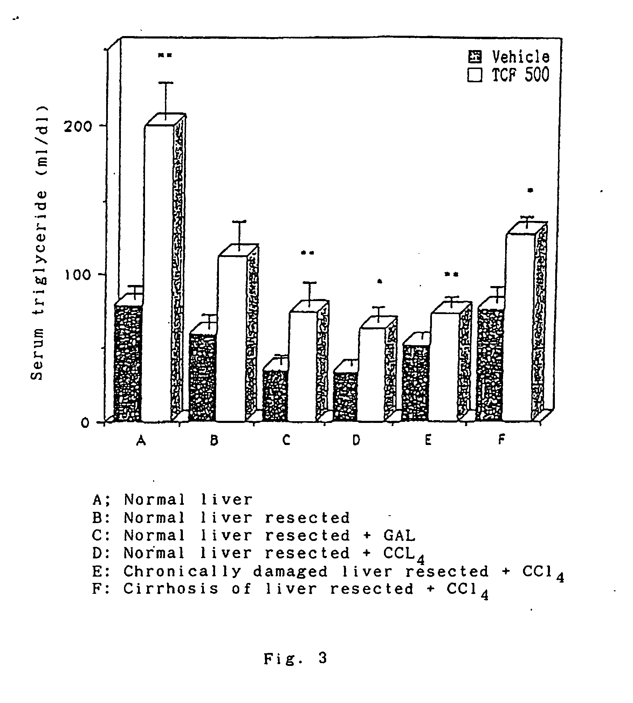

Fig. 3 shows the concentration of serum triglyceride in liver resected pathologic rats.

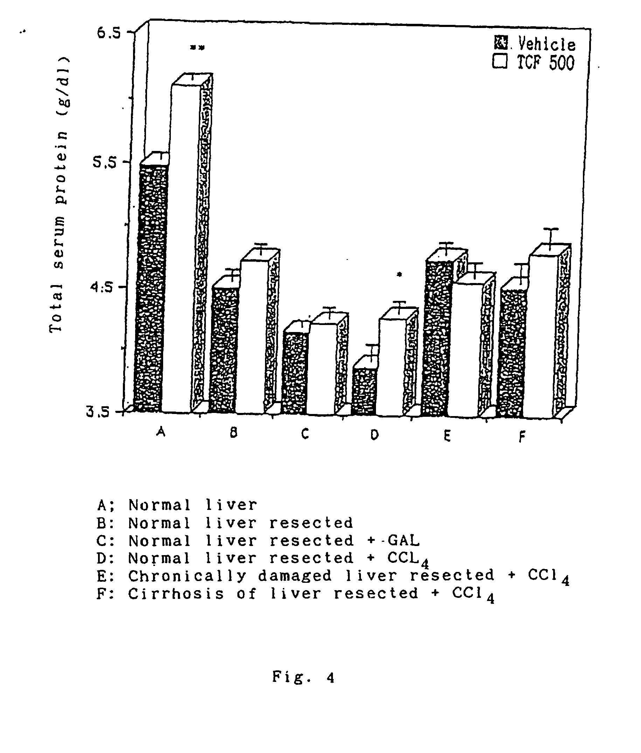

Fig. 4 shows the content of total serum protein in liver resected pathologic rats.

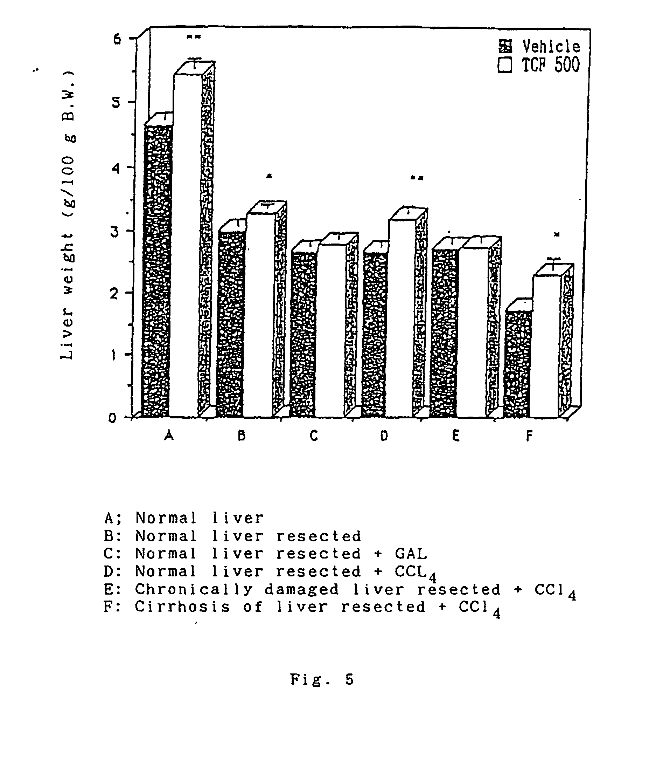

Fig. 5 shows the weight of liver in liver resected pathologic rats.

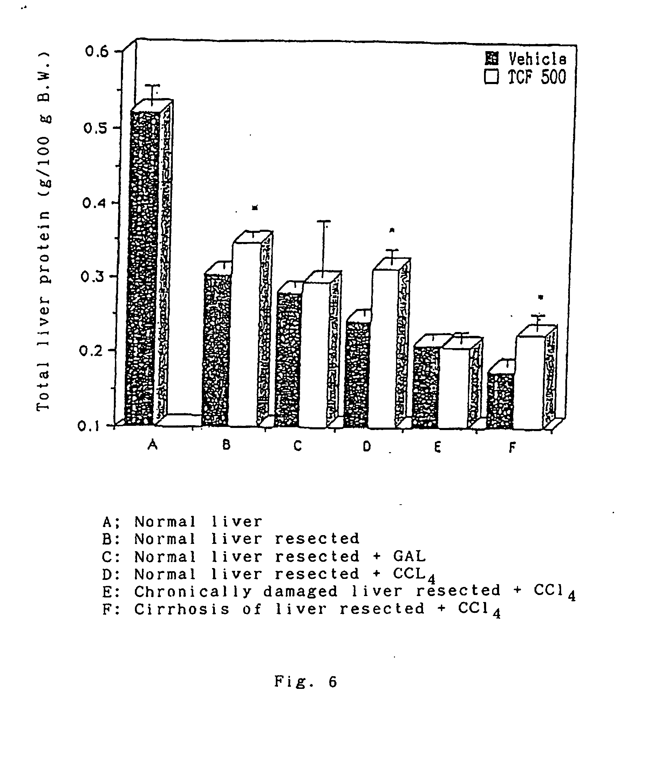

Fig. 6 shows the content of liver protein in liver resected pathologic rats.

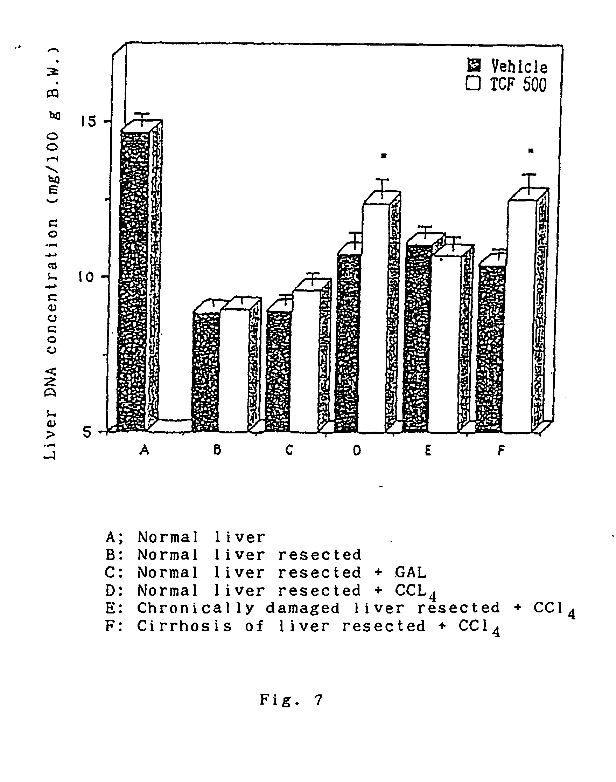

Fig. 7 shows the content of liver DNA in liver resected pathologic rats.
Fig. 8 shows the concentration of total serum protein in liver resected and intermittently or continuously TCF-II administered rats.
Fig. 9 shows the concentration of serum albumin in liver resected and intermittently or continuously TCF-II administered rats.
Fig. 10 shows the concentration of serum HDL-cholesterol in liver resected and intermittently or continuously TCF-II administered rats.
Fig. 11 shows the concentration of serum triglycerides in liver resected and intermittently or continuously TCF-II administered rats.
Fig. 12 shows the regeneration rate of liver resected and intermittently or continuously TCF-II administered rats.
Fig. 13 shows the thrombotest value in liver resected and intermittently or continuously TCF-II administered rats.

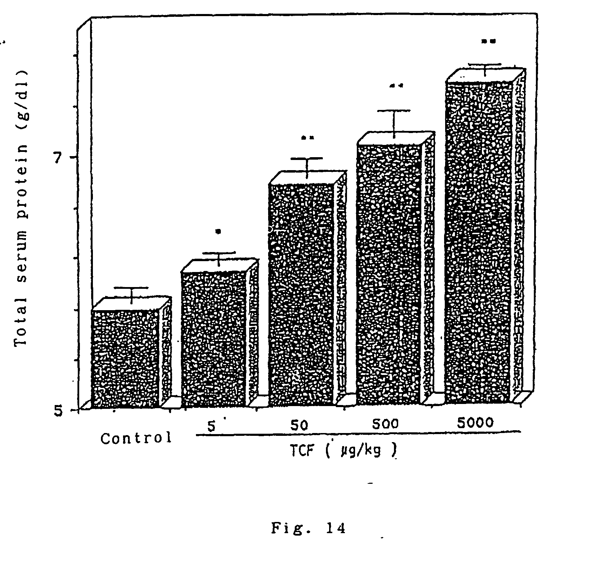

Fig. 14 shows the increase of total serum protein in normal rats administered with the treatment agent of the present invention.

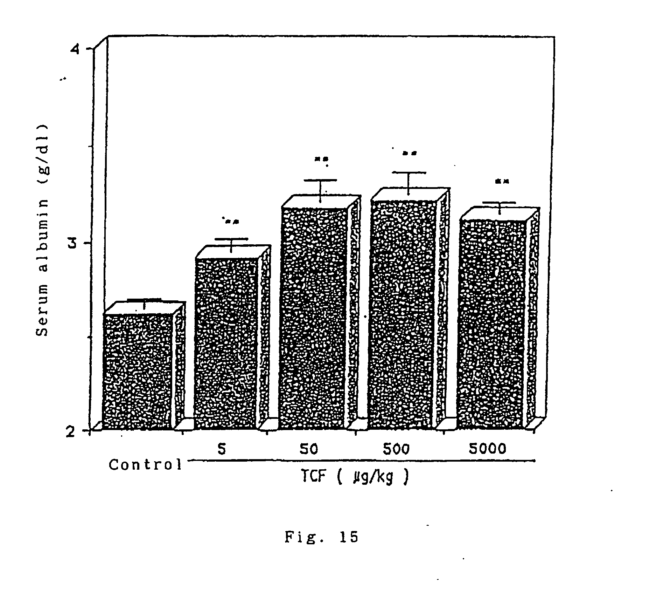

Fig. 15 shows the increase of serum albumin in normal rats administered with the treatment agent of the present invention.

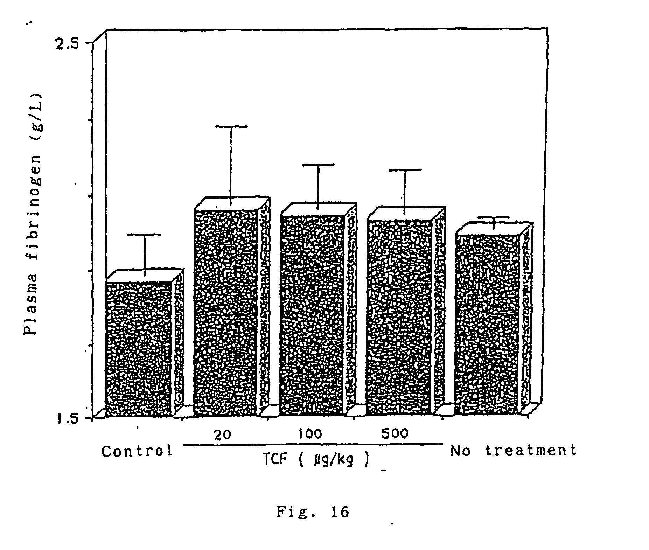

Fig. 16 shows the increase of total plasma fibrinogen in hypoproteinemia rats after 70% liver resection followed by administration of the treatment agent of the present invention.

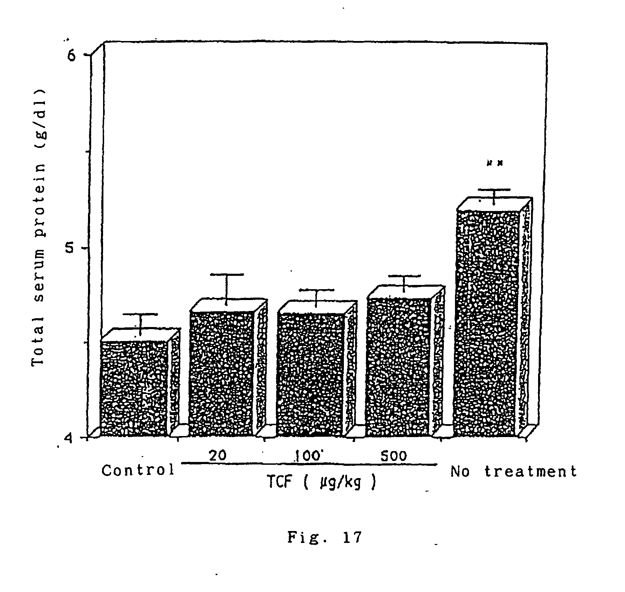

Fig. 17 shows the increase of total serum protein in hypoproteinemia rats after 70% liver resection followed by administration of the treatment agent of the present invention.

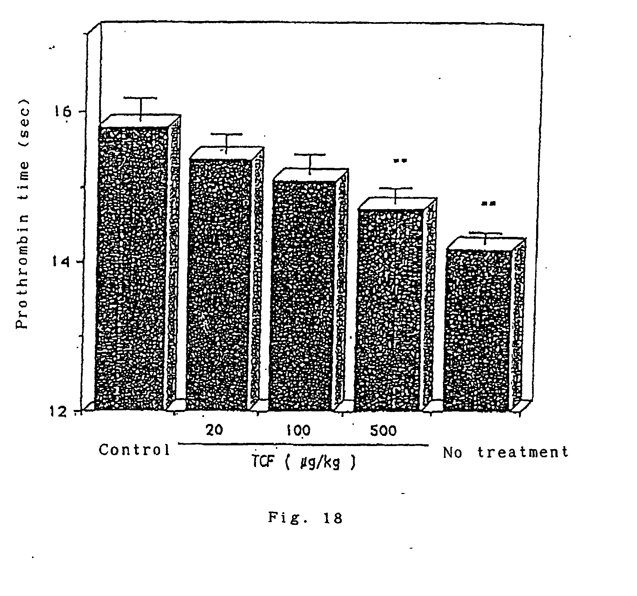

Fig. 18 shows the shortening of prothrombin time in hypoproteinemia rats after 70% liver resection followed by administration of the treatment agent of the present invention.

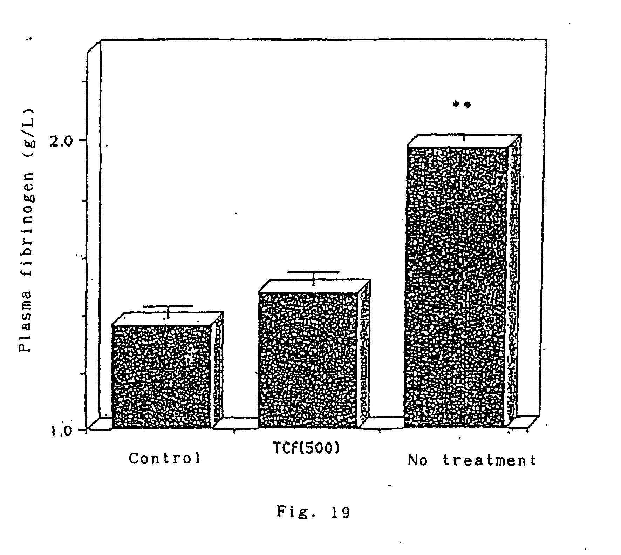

Fig. 19 shows the increase of plasma fibrinogen in hypoproteinemia rats due to DIC followed by administration of the treatment agent of the present invention.

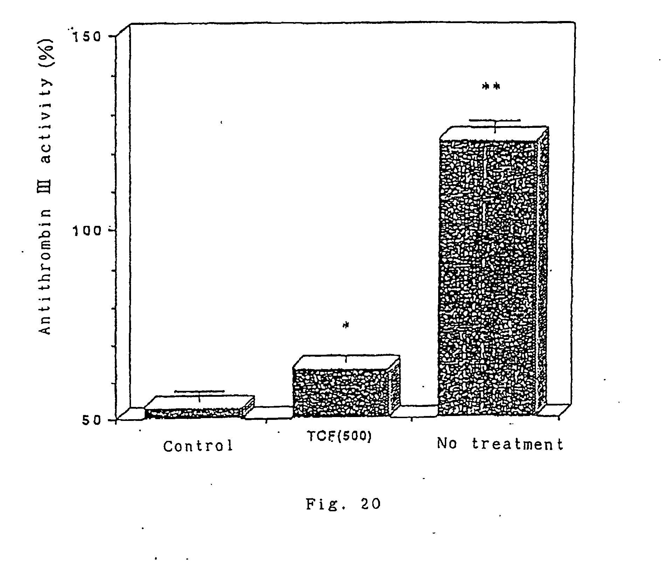

Fig. 20 shows the increase of antithrombin III in hypoproteinemia rats caused by DIC followed by administration of the treatment agent of the present invention.

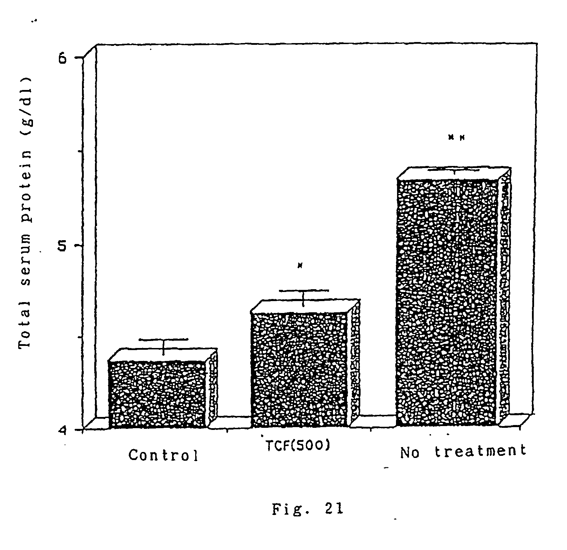

Fig. 21 shows the increase of total serum protein in hypoproteinemia rats caused by - DIC followed by administration of the treatment agent of the present invention.

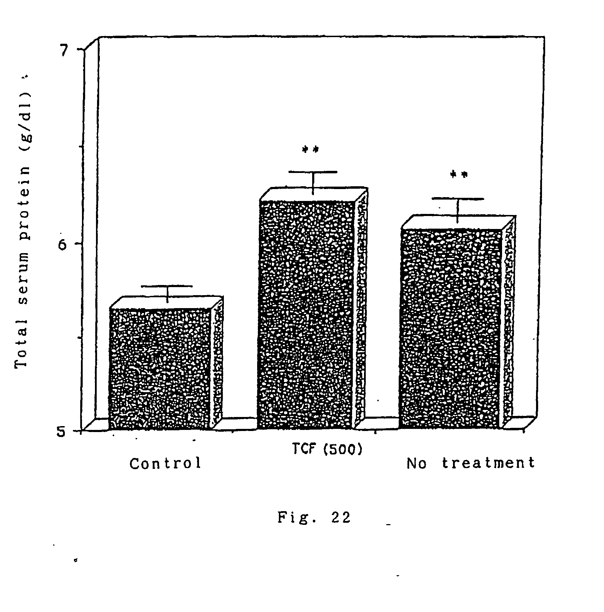

Fig. 22 shows the increase of total serum protein in hypoproteinemia rats caused by chronic renal failure followed by administration of the treatment agent of the present invention. ,

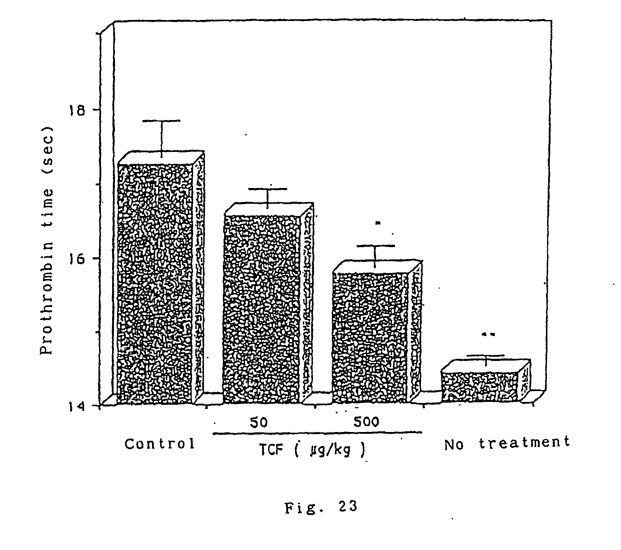

Fig. 23 shows the shortening of prothrombin time in hypoproteinemia rats caused by undernutrition followed by administration of the treatment agent of the present invention.

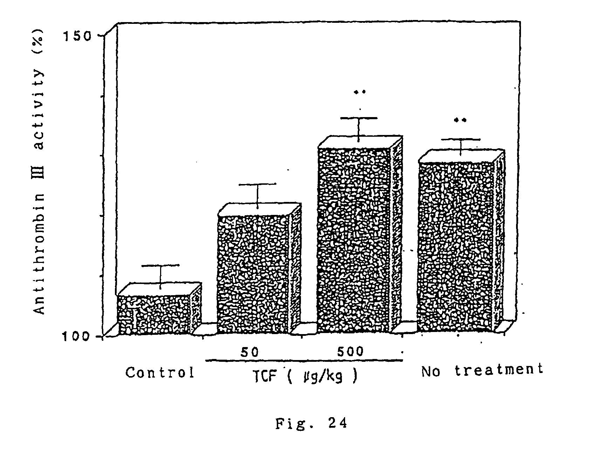

Fig. 24 shows the increase of antithrombin III activity in hypoproteinemia rats caused by undernutrition followed by administration of the treatment agent of the present invention.

In the Figs., * indicates P<0.05 and ** indicates P<0.01.

### Detailed Explanation of Preferred Embodiments

The effective ingredient of the present invention is a known glycoprotein (TCF-II) derived from human fibroblast cells as described previously.

TCF-II showed a molecular weight of 78,000±2,000 or 74,000±2,000 in the non-reduced state, and a common band A of 52,000±2,000 and two bands B of 30,000±2,000 and C of 26,000±2,000 in the reduced state by SDS electrophoresis. TCF-II also showed an isoelectric point at pH 7.4-8.6 and was determined as a glycoprotein having a 723 amino acid sequence.

The above mentioned TCF-II can be obtained by evaporation of a human fibroblast cell culture solution, adsorption in an ion exchange resin and affinity chromatography of the eluate (WO90/10651) or by a genetic engineering method (WO92/01053).

TCF-II can be obtained from human fibroblast cells cultured by the method disclosed in WO90/10651. Furthermore, TCF-II produced by a genetic recombination technique using microorganisms or other cells by the gene sequence disclosed in the above mentioned patent publication may be used. The production of TCF-II by the genetic engineering method may be carried out by the method invented by the present inventors and disclosed in WO92/01053. In addition, TCF-II analogues having different sugar chains or no sugar moieties produced by different host cells or microorganisms may also be used. However, presence of sugar moieties is preferable because of their participation in the in vivo metabolic rate.

TCF-II can be concentrated and purified by conventional isolation and purification methods, for example, precipitation with an organic solvent, salting out, gel filtration chromatography, affinity chromatography using a monoclonal antibody and electrophoresis. The purification by affinity chromatography using a monoclonal antibody disclosed in Japanese Patent Application No. 177236 (1991) by the present inventors may be applied.

The resultant purified TCF-II may be kept under lyophilization or deep freezing.

The liver disease treatment agent of the present invention may be intravenously, intraarterially, intramuscularly or subcutaneously administered as injection preparations. Drugs used for the treatment of liver diseases such as amino acids, vitamins, phospholipids, malotilate, prednisolone and glycyrrhizin may be used concurrently.

Furthermore, the compound of the present invention may be administered as injection preparations and any route such as intravenous, intraarterial, intramuscular and subcutaneous injections can be selected.

The injection preparations of TCF-II may be used singly or in combination with medicines and adjuvants such as human serum albumin, surface active agents, amino acids and sugars.

The doses of TCF-II included in the liver disease treatment agents may be determined according to the symptoms, conditions and age of the patients but preparations containing 100-30,000 µg, preferably 500-3,000 µg of TCF-II are generally administered 1-7 times a week. Chronic administration may be appropriate according to the symptoms and conditions of the patients.

The present invention will be explained in more detail by the following examples.

### Example 1.

### Purification of TCF-II

Purified TCF-II was obtained by cell culture according to the method disclosed in WO90/10651 or Higashio, K. et al. (B.B.R.C., 170, 397-404, 1990).

Human fibroblast cells IMR-90 (ATCC CCL 186), 3 x 10⁶ cells, were inoculated in 100 ml of DMEM medium containing 5% bovine serum in a roller bottle and cultured at rotations of 0.5-2/min. for seven days. The culture was continued up to 1 x 10⁷ cells in total, the proliferated cells were separated by treatment with trypsin and collected at the bottom of the bottle. In the bottle, 100 g of sterilized 5-9 mesh ceramic (Toshiba Ceramic Co., Ltd.) was placed and cultured for 24 hrs. upon standing. Then, 500 ml of the culture medium shown above was added to the bottle and cultured further. The total culture medium was recovered every 7-10 days and fresh culture medium was supplied for further culture. Thus, the culture was continued for two months and four 1/bottle of the culture solution was recovered.

The combined culture solution showed specific activity of 32 µ/ml.

Ultrafiltration of 750 l of the cultured solution was performed using a membrane filter (Amicon Corp., MW 6,000 cut) and the filtrate was chromatographed in five steps using CM Sephadex C-50 (Farmacia Biosystems Corp.), ConA Sepharose (Farmacia Biosystems Corp.), MonoS column (Farmacia Biosystems Corp.) and heparin Sepharose (Farmacia Biosystems Corp.) to give purified TCF-II having specific activity of 5,248,000 U/mg.

### Example 2

### Production of gene recombinant TCF-II

TCF-II gene recombinant cells were cultured according to the method disclosed in WO92/01053 and purified TCF-II was obtained. Transformed Namalwa cells were cultured and 20 1 of the culture solution was obtained. The culture solution was treated successively with HPLC using CM-Sephadex C-50 chromato column, Con-A Sepharose CL-6B chromato column and MonoS column to give approximately 11 mg of active TCF-II.

### Example 3

### Production of pharmaceutical compositions of TCF-II

In the present examples, recombinant TCF-II obtained by Example 2 was used for the production of intravenous, subcutaneous and intramuscular injection preparations.

| | | |
|---|---|---|
| (1) | TCF-II | 40 µg |
| | Human serum albumin | 1 mg |

The above composition was dissolved in 0.01M PBS at pH 7.0 and adjusted to 20 ml in total. The solution was sterilized, divided into vials (2 ml each), lyophilized and sealed.

| | | |
|---|---|---|
| (2) | TCF-II | 40 µg |
| | Tween 80 | 1 mg |
| | Human serum albumin | 100 mg |

This composition was dissolved in a saline solution for injection and adjusted to 20 ml in total. The solution was sterilized, divided into vials (2 ml each), lyophilized and sealed.

| | | |
|---|---|---|
| (3) | TCF- II | 20 µg |
| | Tween 80 | 2 mg |
| | Sorbitol | 4 g |

This composition was dissolved in 0.01M PBS at pH 7.0 and adjusted to 20 ml in total. The solution was sterilized, divided into vials (2 ml each), lyophilized and sealed.

| | | |
|---|---|---|
| (4) | TCF-II | 40 µg |
| | Tween 80 | 2 mg |
| | Glycine | 2 g |

This composition was dissolved in a saline solution for injection and adjusted to 20 ml in total. The solution was sterilized, divided into vials (2 ml each), lyophilized and sealed.

| | | |
|---|---|---|
| (5) | TCF- II | 40 µg |
| | Tween 80 | 1 mg |
| | Sorbitol | 2 g |
| | Glycine | 1 g |

This composition was dissolved in a saline solution for injection and adjusted to 20 ml in total. The solution was sterilized, divided into vials (2 ml each), lyophilized and sealed.

| | | |
|---|---|---|
| (6) | TCF-II | 20 µg |
| | Sorbitol | 4 g |
| | Human serum albumin | 50 mg |

This composition was dissolved in 0.01M PBS at pH 7.0 and adjusted to 20 ml in total. The solution was sterilized, divided into vials (2 ml each), lyophilized and sealed.

| | | |
|---|---|---|
| (7) | TCF- II | 40 µg. |
| | Glycine | 2 g |
| | Human serum albumin | 50 mg |

This composition was dissolved in a saline solution for injection and adjusted to 20 ml in total. The solution was sterilized, divided into vials (2 ml each), lyophilized and sealed.

| | | |
|---|---|---|
| (8) | TCF-II | 10 mg |
| | Human serum albumin | 100 mg |

This composition was dissolved in 0.01M PBS at pH 7.0 and adjusted to 20 ml in total. The solution was sterilized, divided into vials (2 ml each), lyophilized and sealed.

These pharmaceutical preparations can be used as liver disease treatment agents according to the above mentioned dosage and regimen.

Liver disease treatment agents containing TCF-II as an effective ingredient are provided by the present invention. Hereinafter, test experiments with treatment agents prepared according to the present invention will be shown to confirm the therapeutic effects and explain the present invention.

### Experiment 1

### Effect on fatty liver

### (1) Method

To seven week old male Wistar rats, 250 mg/kg of DL-ethionine was intraperitoneally administered successively for four days (n = 10). TCF-II was intravenously administered to these rats at doses of 50 and 500 µg/kg every 12 hrs. and prothrombin time (pT), antithrombin III activity (AT III), blood urea nitrogen (BUN), total cholesterol (T-CHO), phospholipid (PL), HDL cholesterol (HDL) and mitotoxic index to 1,000 hepatocytes after 48 hrs., and total serum protein (TP), transaminase (GOT) and lactic acid dehydrogenase (LDH) after 72 hrs. were determined.

### (2) Results

The average values and their standard errors are shown in Table 1. TCF-II dose dependently improved symptoms of ethionine induced fatty liver rats at doses of 50 µg/kg or over.

**Table 1 TCF-II administered hepatic parameters in ethionine induced fatty liver rats**

| | Normal group | Solvent | 50 µg/kg | 500 µg/kg |
|---|---|---|---|---|
| After | | | | |
| 48 hrs. | | | | |
| PT | 14.13 | 17.28 | 16.57 | 15.78 |
| (sec) | ±0.13** | ±0.48 | ±0.27 | ±0.28** |
| FIB | 1.83 | 1.79 | 2.12 | 2.25 |
| (g/L) | ±0.03 | ±0.11 | ±0.05** | ±0.05** |
| TT | 23.58 | 32.34 | 30.00 | 28.16 |
| (sec.) | ±0.28** | ±1.66 | ±0.76 | ±0.75* |
| AT III | 128.7 | 106.7 | 120 | 131.1 |
| (%) | ±2.5** | ±4.0 | ±4.1* | ±3.8** |
| UN | 18.7 | 30.6 | 30.8 | 23.9 |
| (mg/dl) | ±0.4** | ±3.2 | ±3.0 | ±0.9** |
| T-CHO | 78.1 | 57.3 | 73.2 | 96.2 |
| (mg/dl) | ±2.9** | ±3.9 | ±4.1* | ±6.7** |
| PL | 152.1 | 134.0 | 157.4 | 188.0 |
| (mg/dl) | ±4.3* | ±8.3 | ±4.4** | ±0.6 ** |
| HDL | 39.6 | 15.3 | 18.6 | 23.1 |
| (mg/dl) | ±1.5 | ±2.1 | ±2.5 | ±3.1 |
| M.I. | 5.2 | 0.1 | 0.4 | 2.9 |
| (/1,000 cells) | ±1.1** | ±0.1 | ±0.2 | ±0.9* |
| Liver | 4.85 | 4.66 | 4.89 | 4.84 |
| weight | ±0.09 | ±0.18 | ±0.08 | ±0.08 |
| (g/100 g B.W.) | | | | |
| After | | | | |
| 72 hrs. | | | | |
| TP | 5.81 | 5.77 | 6.08 | 6.23 |
| (mg/dl) | ±0.08** | ±0.09 | ±0.08 | ±0.08** |
| GOT | 68.3 | 93.9 | 77.2 | 60.1 |
| (IU/L) | ±2.8* | ±5.7 | ±3.3 | ±2.3** |
| LDH | 1499 | 2605 | 1985 | 1400 |
| (IU/L) | ±190* | ±256 | ±126 | ±121** |

| | | | | |
|---|---|---|---|---|
| **: Significant at P=0.01 to solvent group *: Significant at P=0.05 to solvent group | | | | |

### Experiment 2

### Effect on cholestatic liver damage

### (1) Method

To seven week old male Wistar rats, 50 mg/kg of α-naphthyl isothiocyanate was orally administered. TCF-II was intravenously administered twice to these rats immediately after the administration of α-naphthyl isothiocyanate and after 12 hrs. Blood was drawn 12 hrs. after the administration of TCF-II and serum transaminase (GOT, GPT), alkaline phosphatase (ALP), γ-glutamyl transpeptidase (*γ-*GTP), total bilirubin (T-BIL), direct bilirubin (D-BIL) and the excretion of foreign materials by liver (BSP test) were measured.

### (2) Results

The average values and standard errors are shown in Table 2. TCF-II dose dependently improved all parameters at doses of 500 µ g/kg.

**Table 2 TCF-II administered hepatic parameters in cholestatic damaged liver rats**

| | Normal group | Solvent | 50 µg/kg | 500 µg/kg |
|---|---|---|---|---|
| GOT(IU/L) | 71.2 | 230.1 | 211.9 | 183.7 |
| | ±3.1** | ±25.6 | ±18.9 | ±26.3 |
| GPT(IU/L) | 24.6 | 62.9 | 56.6 | 48.0 |
| | ±1.1** | ±8.0 | ±5.4 | ±7.8 |
| ALP(IU/L) | 609.7 | 1034.7 | 1088.8 | 839.8 |
| | ±35.0** | ±63.6 | ±77.3 | ±70.0 |
| γ-GTP | 2.2 | 11.5 | 12.2 | 8.3 |
| (IU/L) | ±0.2** | ±0.8 | ±0.9 | ±0.7** |
| T-Bil | 0.22 | 2.05 | 2.09 | 1.60 |
| (mg/dl) | ±0.11** | ±0.1 | ±0.1 | ±0.12* |
| D-Bil | 0.15 | 1.18 | 1.19 | 0.93 |
| (mg/dl) | ±0.01** | ±0.0 | ±0.0 | ±0.08* |
| BSP | 0.50 | 29.38 | - | 24.76 |
| retention | ±0.02** | ±0.51 | - | ±1.87** |
| rate (%) | | | | |

| | | | | |
|---|---|---|---|---|
| **: Significant at P=0.01 to solvent group *: Significant at P=0.05 to solvent group | | | | |

### Experiment 3

### Therapeutic effect according to type of administration

### (1) Method

Seven week old male Wistar rats, body weight 200 g, were used for the experiment and normal liver rat group was prepared by resection of 70% of the liver. The administration of TCF-II was started immediately after the resection intravenously and intermittently at 12 hr. intervals (n = 6) or by continuous infusion (n = 12) and the responses were compared. Both groups were administered at the same dose of one mg/kg/day. Total serum protein, albumin, triglyceride, HDL-cholesterol and thrombotest value 48 hrs. after the resection were determined and liver weight was determined to estimate the regeneration rate of the liver.

### (2) Results

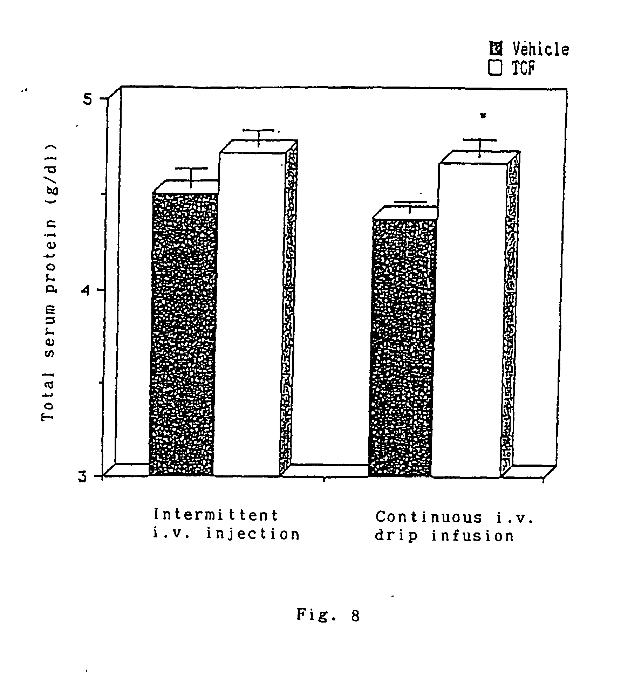

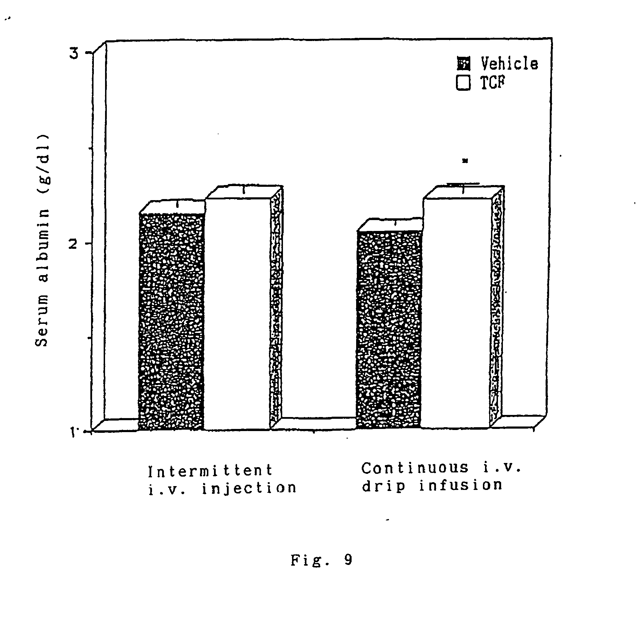

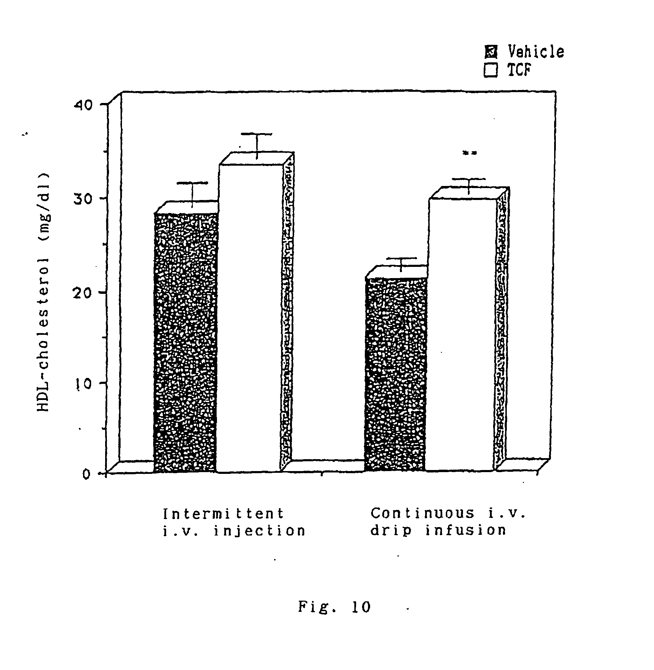

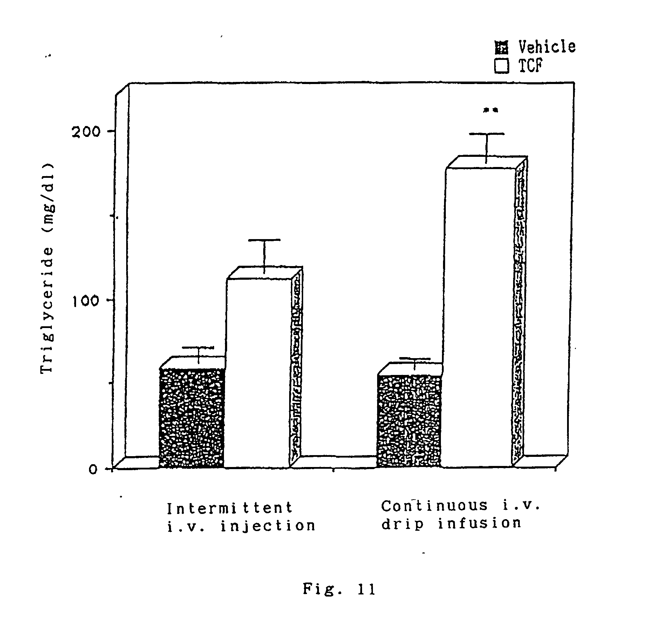

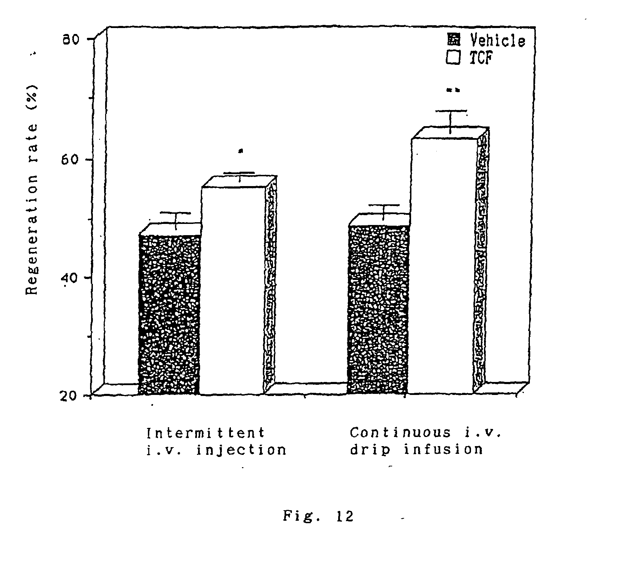

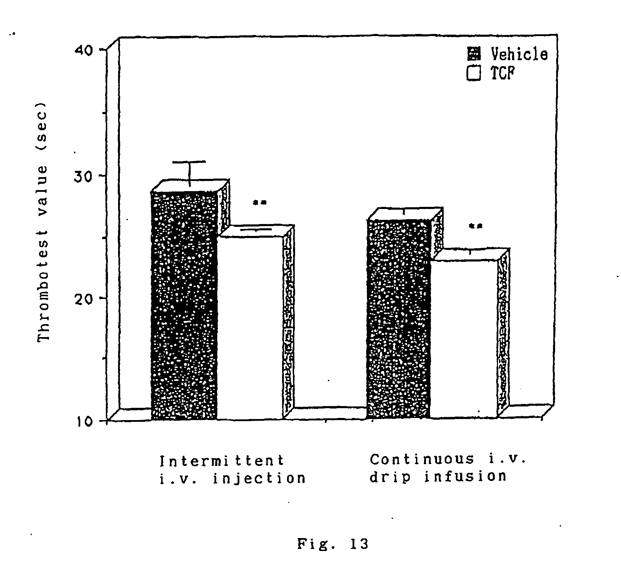

Changes of parameters by the administration of TCF-II in 70% liver resected rats are shown in total serum protein (Fig. 8), albumin (Fig. 9), HDL-cholesterol (Fig. 10), triglyceride (Fig. 11), regeneration rate (Fig. 12) and thrombotest value (Fig. 13). All parameters showed improvement in the continuous drip infusion group.

Above results of experiments exhibit excellent effect of the agent of the present invention against heretofore intractable liver diseases.

## Claims

1. Use of TCF-II in the preparation of a medicament for the treatment of fatty liver or cholestatic liver diseases.

2. The use according to claim 1 in which the medicament is in injectable form.

## Patentansprüche

1. Verwendung von TCF-II bei der Herstellung eines Medikaments für die Behandlung von Fettleber oder cholestatischen Lebererkrankungen.

2. Verwendung nach Anspruch 1, bei der das Medikament in injizierbarer Form vorliegt.

## Revendications

1. Utilisation de TCF-II dans la préparation d'un médicament pour le traitement des maladies de stéatose hépatique ou de foie cholestatique.

2. Utilisation selon la revendication 1, dans laquelle le médicament se présente sous forme injectable.
